# EUROPEAN PATENT APPLICATION

(11) **EP 4 765 130 A1**
(43) Date of publication of application: **24.06.2026**
(21) Application number: 24853845.6
(22) Date of filing: 15.08.2024
(51) Int. Cl.: G16B 20/50

(54) **PREDICTION FOR INFLUENCE OF MUTATION ON PROTEASE ACTIVITY**

(30) Priority: 15.08.2023 CN 202311028534
(71) Applicant: Genscript (Shanghai) Biotech Co., Ltd., Shanghai 200131 (CN); Nanjing GenScript Biotech Co., Ltd., Nanjing, Jiangsu 211100 (CN)
(72) Inventor: LI, Gen, Shanghai 200131 (CN); FAN, Long, Shanghai 200131 (CN); ZHANG, Ning, Nanjing, Jiangsu 211100 (CN)
(74) Representative: Ström & Gulliksson AB
(86) International application number: PCT/CN2024/112259
(87) International publication number: WO 2025/036438

(57) **Abstract**

The present disclosure relates to a prediction for the influence of a mutation on protease activity. A method for predicting the influence of a mutation on protease activity comprises: on the basis of sequence information of a protease sample to be subjected to prediction, performing feature extraction by using a pre-training model, so as to obtain a sequence feature of said protease sample (S110); on the basis of three-dimensional structure information of said protease sample, obtaining a structural feature of said protease sample (S120); and on the basis of the sequence feature and the structural feature, performing prediction by using a prediction model based on a graph neural network, and determining an activity change prediction result after said protease sample mutates (S130). Further disclosed is a method for constructing a prediction model used for predicting the influence of a mutation on protease activity. By means of the method in the present disclosure, prediction is performed on the basis of an advanced natural language processing model and a graph neural network, in combination with a sequence and a three dimensional structure, and by means of an artificial intelligence model, showing a performance superior to that of the other existing methods, and proving the wide application prospects of the method in protease engineering.

## Description

### CROSS-REFERENCE TO RELATED APPLICATIONS

The present application claims priority to Chinese Patent Application No. 202311028534.0 entitled "PREDICTION FOR INFLUENCE OF MUTATION ON PROTEASE ACTIVITY" and filed with the China National Intellectual Property Administration on August 15, 2023, which is incorporated herein by reference in its entirety.

### TECHNICAL FIELD

The present disclosure relates to protease optimisation, and in particular, relates to predicting the influence of an amino acid mutation in a protease on protease activity.

### BACKGROUND

Enzymes are an important type of biocatalyst. They have broad application prospects in industrial biocatalysis, particularly in the production of pharmaceutical intermediates, due to their high selectivity, biocompatibility, and mild reaction conditions. Most enzymes are proteins, and the molecular structure and function of proteins are influenced by factors such as temperature, pH, and activators. Therefore, the catalytic activity of proteases can only be exhibited under specific conditions, and engineering modification of proteases for catalytic activity is usually required to adapt to different production environments and functional requirements.

Directed evolution and rational design are two common strategies for protease optimisation. The directed evolution strategy has been successfully applied to the modification of protease properties such as activity, stability, substrate specificity, and stereoselectivity. For this achievement, American scientist Frances H. Arnold was awarded the 2018 Nobel Prize in Chemistry. However, directed evolution requires the construction of large-scale mutant libraries and the establishment of high-throughput screening methods, which consume substantial human, material, and financial resources. The directed evolution method also faces difficulties in achieving comprehensive search of sequence space, and thus has inherent defects. The rational design strategy relies on understanding of the relationship between the structure and function of a protease to predict potential mutants, and followed by constructing these mutants in target genes via site-directed mutagenesis. Essentially, compared to directed evolution, rational design offers higher efficiency in protease modification. In addition, the rational design method is universal, and an effective rational design strategy can be universally applied to the modification of various proteases. However, currently the accuracy of the rational design methods is generally low, and their application scope is far less extensive than that of directed evolution. In recent years, artificial intelligence-assisted protein engineering has gradually evolved into an efficient new strategy for protein design. It has demonstrated unique advantages in multiple aspects such as protein structure prediction, function prediction, stability prediction, and antibody affinity prediction, emerging as another technological wave following rational design and directed evolution.

In recent years, with the rapid development of computer technology, various neural network models have been rapidly updating and iterating toward a more accurate and efficient direction, and artificial intelligence (AI)-assisted enzyme engineering has gradually evolved into an efficient new strategy for protease design. In order to address the explosive growth of protein sequence databases driven by large-scale gene sequencing, the parameter size of the current largest protein language model has reached 15 billion (see Lin, Zeming, et al., 2023, "Evolutionary-scale Prediction of Atomic-level Protein Structure with a Language Model", Science 379(6637): 1123-1130, which is incorporated herein by reference in its entirety and made a part of the present disclosure). The continuous development of high-performance computing and advancements in natural language processing (NLP) have enabled researchers to leverage large protein databases to enhance the prediction of sequence properties or annotations from relatively small experimental datasets.

After decades of development, dozens of AI-based methods for protein stability prediction have emerged, with performance surpassing that of traditional rational design methods. Unlike protease stability prediction, SCANEER (Sequence Co-evolutionary Analysis to Control Efficiency of Enzyme Reactions) is currently the only method that leverages sequence co-evolutionary analysis to discover evolutionarily feasible alternative amino acids for enhancing protease activity (see Kim, Donghyo et al., 2022, "Enzyme activity engineering based on sequence co-evolution analysis", Metabolic Engineering 74: 49-60, which is incorporated herein by reference in its entirety and made a part of the present disclosure). SCANEER evaluates the co-evolutionary relationships between amino acid pairs in multiple sequence alignment (MSA). Amino acid pairs, i.e., amino acid substitutions, that are not observed or rarely observed in MSA cannot be predicted, ultimately resulting in predictable mutations for each protease accounting for only 47% of all possible mutations, which greatly limits the application scope of this method. A gap persists in the application of state-of-the-art AI techniques to protease activity prediction.

Currently, applying natural language processing to protease activity prediction faces numerous issues. First, as artificial intelligence algorithms rely heavily on data, the quantity and quality of initial data determine the generalization performance of the trained models (see Usmanova, Dinara R., et al., 2018, "Self-consistency Test Reveals Systematic Bias in Programs for Prediction Change of Stability upon Mutation", Bioinformatics 34(21): 3653-3658, which is incorporated herein by reference in its entirety and made a part of the present disclosure). Insufficient sample size or poor dataset quality will lead to overfitting or underfitting in the model, which may be a key factor that may limit AI-based protease activity prediction. Meanwhile, biases in protease evolution and experiments lead to dataset imbalance, which in turn impairs the performance of prediction models. Second, for different substrates under different experimental conditions such as temperature and pH, the same mutation exerts different influences on protease activity. Such multi-label data also brings about significant difficulties to prediction tasks.

### SUMMARY

Stability and catalytic activity are the two most important properties of proteases. Stability has been extensively studied, with dozens of AI-based methods developed to predict the influence of mutations on protease stability, demonstrating excellent performance. In contrast, catalytic activity prediction has stagnated due to limitations in datasets and methodologies. To date, only the SCANEER method (cited above) can predict the influence of single-point mutations on protease activity. However, this method is based on co-evolutionary information, and therefore can only predict mutations that have occurred in the evolutionary process, posing significant limitations and thus failing to be widely applied. On the basis of the defects existing in the current methods for predicting the influence of the mutation on protease activity as mentioned above, the present disclosure proposes a method for predicting the influence of a mutation on protease activity. This is a deep learning method based on a novel natural language processing method and graph-structured data, in which a T5 (i.e., Transfer Text-to-Text Transformer) pre-trained model is used to extract sequence features, and a graph neural network model (e.g., Graph Attention Network (GAT)) is used to extract structural features. The method successfully applies protein language models and graph attention network models to predict the changes in protease activity caused by single-point missense mutations.

According to a first aspect of the present disclosure, provided is a method for predicting the influence of a mutation on protease activity. The method may include the following steps: performing feature extraction using a pre-trained model on the basis of sequence information of a protease sample to be predicted, so as to obtain a sequence feature of the protease sample to be predicted; obtaining a structural feature of the protease sample to be predicted on the basis of three-dimensional structural information of the protease sample to be predicted; and performing prediction using a graph neural network-based prediction model on the basis of the sequence feature and the structural feature, so as to determine a prediction result for activity change of the protease sample to be predicted after mutation.

In the method according to the first aspect of the present disclosure, the sequence information of the protease sample to be predicted may include amino acid sequence information of the protease sample to be predicted before mutation and after mutation.

In another aspect, the sequence information of the protease sample to be predicted may include amino acid sequence information of the protease sample to be predicted before mutation and specified mutation information. Thus, amino acid sequence information of the protease sample to be predicted after mutation can be obtained on the basis of the amino acid sequence information of the protease sample to be predicted before mutation and the specified mutation information.

Preferably, the specified mutation information includes a specified mutation site and/or a specified amino acid type after mutation.

In the method according to the first aspect of the present disclosure, said performing feature extraction using the pre-trained model on the basis of the sequence information of the protease sample to be predicted, so as to obtain the sequence feature of the protease sample to be predicted, may further include: performing feature extraction separately on the amino acid sequence information of the protease sample to be predicted before mutation and after mutation using the pre-trained model, so as to obtain sequence feature information before mutation and sequence feature information after mutation; and concatenating the sequence feature information before mutation and the sequence feature information after mutation, so as to obtain the sequence feature of the protease sample to be predicted.

In the method according to the first aspect of the present disclosure, the pre-trained model may be implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

In the method according to the first aspect of the present disclosure, the three-dimensional structural information of the protease sample to be predicted may include the three-dimensional structural information of the protease sample to be predicted obtained through at least one of the following databases or prediction software: PDB database, AlphaFold2, I-TASSER, RoseTTAFold, Modeller, and Swiss-model.

In the method according to the first aspect of the present disclosure, the three-dimensional structural information of the protease sample to be predicted may include an interaction network, a secondary structure, an amino acid residue distance, or a physical environment; preferably, the three-dimensional structural information of the protease sample to be predicted includes an interaction network.

Preferably, the three-dimensional structural information of the protease sample to be predicted is acquired by: acquiring coordinates of a three-dimensional structure of the protease sample to be predicted; calculating distances between amino acid residue pairs in the three-dimensional structure of the protease on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire protease sample to be predicted.

Correspondingly, in the method according to the first aspect of the present disclosure, said obtaining the structural feature of the protease sample to be predicted on the basis of the three-dimensional structural information of the protease sample to be predicted, may include: taking a mutation site as the centre, extracting an adjacency matrix of a specified size from the adjacency matrix of the entire protease sample to be predicted, which is used as the structural feature of the protease sample to be predicted. In some implementations, taking a mutation site as the centre, an N*N adjacency matrix (where N is an integer greater than or equal to 1) is extracted from the adjacency matrix of the entire protease sample to be predicted. Said extracting the N*N adjacency matrix from the adjacency matrix of the entire protease sample to be predicted can be performed on the basis of sequences on the left and right sides of the mutation site or on the basis of distances from the mutation site. In an implementation, taking a mutation site as the centre, an amino acid within a distance of less than 10 angstroms from the mutation site is selected from the adjacency matrix of the entire protease sample to be predicted, which is used as a structural feature of the protease sample to be predicted.

In the method according to the first aspect of the present disclosure, preferably, said performing prediction using the graph neural network-based prediction model on the basis of the sequence feature and the structural feature, so as to determine the prediction result for activity change of the protease sample to be predicted after mutation, may further include: inputting the sequence feature and the structural feature into the graph neural network, so as to obtain a probability feature related to activity change; and combining the probability feature related to activity change with supplementary features from other prediction methods, and inputting the combined features into a decision tree model to finally obtain a classification of activity change.

Preferably, said inputting the sequence feature and the structural feature into the graph neural network, so as to obtain the probability feature related to activity change, may include: inputting the sequence feature and the structural feature into the graph neural network; updating the features through matrix multiplication and aggregation operations using several layers of graph network structures, and then outputting the updated features; merging the features output by the several layers of graph network structures, respectively; compressing the features using a pooling operation; and performing activity change classification on the features on the basis of a classification function of an output layer, and outputting the probability feature related to activity change. Preferably, the pooling operation here may be average pooling, max pooling, or K-max pooling. The probability feature of activity change here may be a probability value of increased activity of the protease sample after mutation.

In the method according to the first aspect of the present disclosure, the graph neural network-based prediction model can be constructed through the steps of: acquiring a training set, where the training set includes information of a plurality of training samples, and the information of each of the training samples includes three-dimensional structural information of a sample protease, amino acid sequence information of the sample protease before mutation and after mutation, and a mutation activity change label; performing feature extraction using a pre-trained model on the basis of the amino acid sequence information of the sample protease before mutation and after mutation, so as to obtain sequence features of the plurality of training samples; obtaining structural features of the plurality of training samples on the basis of the three-dimensional structural information of the sample protease; and training a graph neural network on the basis of the sequence features, the structural features, and the mutation activity change labels, so as to obtain the graph neural network-based prediction model.

Preferably, the graph neural network-based prediction model is a classifier.

Preferably, the graph neural network is a graph convolutional network or a graph attention network.

Preferably, said training the graph neural network on the basis of the sequence features, the structural features, and the mutation activity change labels, so as to obtain the graph neural network-based prediction model, may further include: inputting the sequence features and the structural features into the graph neural network, so as to obtain probability features related to activity change; combining the probability features related to activity change with supplementary features from other prediction methods, and inputting the combined features into a decision tree model to obtain a classification of activity change; and taking the mutation activity change labels as output targets, adjusting parameters of the prediction model.

Preferably, the decision tree model is a gradient boosting decision tree (GBDT) model. More preferably, the GBDT model is implemented using a LightGBM framework.

Preferably, experimental data from a public database can be obtained as the training set. Meanwhile, the size of the training set can be doubled by performing inversion processing on the experimental data from the public database.

According to a second aspect of the present disclosure, provided is a method for constructing a prediction model for predicting the influence of a mutation on protease activity. The method may include the following steps: acquiring a training set, where the training set includes information of a plurality of training samples, and the information of each of the training samples includes three-dimensional structural information of a sample protease, amino acid sequence information of the sample protease before mutation and after mutation, and a mutation activity change label; performing feature extraction using a pre-trained model on the basis of the amino acid sequence information of the sample protease before mutation and after mutation, so as to obtain sequence features of the plurality of training samples; obtaining structural features of the plurality of training samples on the basis of the three-dimensional structural information of the sample protease; and training a graph neural network on the basis of the sequence features, the structural features, and the mutation activity change labels, so as to obtain the prediction model for predicting the influence of the mutation on protease activity.

In the method according to the second aspect of the present disclosure, said performing feature extraction using the pre-trained model on the basis of the amino acid sequence information of the sample protease before mutation and after mutation, so as to obtain the sequence features of the plurality of training samples, may include: performing feature extraction separately on the amino acid sequence information of the sample protease before mutation and after mutation using the pre-trained model, so as to obtain sequence feature information before mutation and sequence feature information after mutation; and concatenating the sequence feature information before mutation and the sequence feature information after mutation, so as to obtain the sequence features of the plurality of training samples.

In the method according to the second aspect of the present disclosure, the pre-trained model may be implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

In the method according to the second aspect of the present disclosure, the three-dimensional structural information of the sample protease may include three-dimensional structural information of the sample protease obtained through at least one of the following databases or prediction software: PDB database, AlphaFold2, I-TASSER, RoseTTAFold, Modeller, and Swiss-model.

In the method according to the second aspect of the present disclosure, the three-dimensional structural information of the sample protease may include an interaction network, a secondary structure, an amino acid residue distance, or a physical environment; preferably, the three-dimensional structural information of the sample protease comprises an interaction network.

Preferably, the three-dimensional structural information of the sample protease may be acquired by: acquiring coordinates of a three-dimensional structure of the sample protease; calculating distances between amino acid residue pairs in the three-dimensional structure of the sample protease on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire sample protease.

Correspondingly, in the method according to the second aspect of the present disclosure, said obtaining the structural features of the plurality of training samples on the basis of the three-dimensional structural information of the sample protease, may include: taking a mutation site as the centre, extracting an adjacency matrix of a specified size from the adjacency matrix of the entire sample protease, which is used as the structural feature of the sample protease. In some implementations, taking a mutation site as the centre, an N*N adjacency matrix (where N is an integer greater than or equal to 1) is extracted from the adjacency matrix of the entire sample protease. Said extracting the N*N adjacency matrix from the adjacency matrix of the entire sample protease can be performed on the basis of sequences on the left and right sides of the mutation site or on the basis of distances from the mutation site. In an implementation, taking a mutation site as the centre, an amino acid within a distance of less than 10 angstroms from the mutation site is selected from the adjacency matrix of the entire sample protease, which is used as a structural feature of the sample protease.

In the method according to the second aspect of the present disclosure, said training the graph neural network on the basis of the sequence features, the structural features, and the mutation activity change labels, so as to obtain the prediction model for predicting the influence of the mutation on protease activity, may further include: inputting the sequence features and the structural features into the graph neural network, so as to obtain probability features related to activity change; combining the probability features related to activity change with supplementary features from other prediction methods, and inputting the combined features into a decision tree model to obtain a classification of activity change; and taking the mutation activity change labels as output targets, adjusting parameters of the prediction model.

Preferably, said inputting the sequence features and the structural features into the graph neural network, so as to obtain the probability features related to activity change, may include: inputting the sequence features and the structural features into the graph neural network; updating the features through matrix multiplication and aggregation operations using several layers of graph network structures, and then outputting the updated features; merging the features output by the several layers of graph network structures, respectively; compressing the features using a pooling operation; and performing activity change classification on the features on the basis of a classification function of an output layer, and outputting the probability features related to activity change. Preferably, the pooling operation may be average pooling, max pooling, or K-max pooling.

Preferably, the prediction model is a classifier.

Preferably, the graph neural network is a graph convolutional network or a graph attention network.

Preferably, the decision tree model is a GBDT model. More preferably, the GBDT model is implemented using a LightGBM framework.

Preferably, experimental data from a public database can be obtained as the training set. Meanwhile, the size of the training set can be doubled by performing inversion processing on the experimental data from the public database.

According to a third aspect of the present disclosure, provided is a system for predicting the influence of a mutation on protease activity. The system may include: an acquisition module, configured to acquire sequence information and three-dimensional structural information of a protease sample to be predicted; and a processing module, configured to obtain a prediction result for activity change of the protease sample to be predicted after mutation by inputting the sequence information and the three-dimensional structural information of the protease sample to be predicted. The processing module further includes the following sub-modules: a sequence feature sub-module, configured to perform feature extraction using a pre-trained model on the basis of the sequence information of the protease sample to be predicted, so as to obtain a sequence feature of the protease sample to be predicted; a structural feature sub-module, configured to obtain a structural feature of the protease sample to be predicted on the basis of the three-dimensional structural information of the protease sample to be predicted; and a model prediction sub-module, configured to perform prediction using a graph neural network-based prediction model on the basis of the sequence feature and the structural feature, so as to determine a prediction result for activity change of the protease sample to be predicted after mutation.

The system for predicting the influence of the mutation on protease activity according to the third aspect of the present disclosure can be implemented by a computer. Preferably, the system can implement the following operations by executing a computer program: acquiring sequence information and three-dimensional structural information of a protease sample to be predicted; obtaining a prediction result for activity change of the protease sample to be predicted after mutation by inputting the sequence information and the three-dimensional structural information of the protease sample to be predicted, where the operation further includes the following sub-operations: performing feature extraction using a pre-trained model on the basis of sequence information of a protease sample to be predicted, so as to obtain a sequence feature of the protease sample to be predicted; obtaining a structural feature of the protease sample to be predicted on the basis of three-dimensional structural information of the protease sample to be predicted; and performing prediction using a graph neural network-based prediction model on the basis of the sequence feature and the structural feature, so as to determine a prediction result for activity change of the protease sample to be predicted after mutation.

According to a fourth aspect of the present disclosure, provided is a non-transitory computer-readable storage medium for storing a computer program. The computer program includes instructions. The instructions, when executed by a processor of an electronic device, cause the electronic device to perform the method for predicting the influence of the mutation on protease activity according to the first aspect of the present disclosure, or the method for constructing the prediction model for predicting the influence of the mutation on protease activity according to the second aspect of the present disclosure.

According to a fifth aspect of the present disclosure, provided is a computer system. The computer system includes: a processor, a memory, and a computer program. The computer program is stored in the memory and configured to be executed by the processor. The computer program includes instructions for performing the method for predicting the influence of the mutation on protease activity according to the first aspect of the present disclosure, or the method for constructing the prediction model for predicting the influence of the mutation on protease activity according to the second aspect of the present disclosure.

A distinctive feature of the above method lies in establishing a connection between the primary sequence and three-dimensional structure of proteases through a graph neural network, particularly a graph attention network (GAT), to further predict mutation effects. Thus, the method may provide novel insights for addressing the annotation challenges posed by the ever-increasing volume of sequence and structural data. To address the issues of low quality and bias in experimental data, in the present disclosure, 5449 data entries containing experimental mutation information are extracted from the D3DistalMutation database, and the dataset is reversed (specific details of the reversal process are described below), which means that the final training dataset contains 10,998 pieces of mutation information and the number of mutations leading to decreased activity and those leading to increased activity are equal. The finally obtained model can not only predict mutations at any position in a specified protease sequence but also achieve performance exceeding expectations on the experimental test set. The method can be combined with methods for predicting protein stability after mutations to address the negative correlation between protease stability and activity, namely the so-called activity-stability trade-off mechanism. The method can not only improve protease stability but also address the issue of potential decline in activity, thus having broad application prospects.

The beneficial technical effects of the present disclosure include:
1. High innovativeness: In the present disclosure, the latest AI method is used to predict the influence of a mutation on protease activity based on a self-constructed dataset, which fills the research gap in the field.
2. Superior performance: As can be seen from the comparison results between the method in the embodiments and the only existing SCANEER below, the method of the present disclosure exhibits superior performance.
3. Broad application prospects: The method can predict the influence of a mutation on protease activity that cannot be predicted by the only existing SCANEER. The portion of mutations that cannot be predicted by SCANEER is more meaningful. This is because the portion of mutations that can be predicted by SCANEER has a high degree of overlap with those identified by the rational design method, and the selected results are likely to be restricted by other patents or literature. In contrast, the method of the present disclosure can identify beneficial mutation sites that are difficult to identify through evolutionary information.

### BRIEF DESCRIPTION OF THE DRAWINGS

The present disclosure will be more fully understood from the following detailed description in conjunction with the accompanying drawings, in which similar elements are numbered in a similar manner.
FIG. 1 is a flow chart of a method for predicting the influence of a mutation on protease activity according to the embodiments of the present disclosure.
FIG. 2 is a joint schematic diagram of a method for predicting the influence of a mutation on protease activity according to the embodiments of the present disclosure, and a method for constructing a prediction model for predicting the influence of a mutation on protease activity according to the embodiments of the present disclosure.
FIG. 3 is a flow chart of a method for constructing a prediction model for predicting the influence of a mutation on protease activity as used in the present disclosure.
FIG. 4 is a schematic diagram of an adjacency matrix.
FIG. 5 is a schematic diagram illustrating the formation of a node feature matrix.
FIG. 6 is a schematic diagram illustrating the structure-based construction of an adjacency matrix.
FIG. 7 is a schematic diagram illustrating the sequence-based construction of a node feature matrix.
FIG. 8 is a schematic diagram of the framework of the prediction model.
FIG. 9 shows a schematic block diagram of a system for predicting the influence of a mutation on protease activity according to the present disclosure.

### DETAILED DESCRIPTION

Unless otherwise stated, technical and scientific terms used herein have the same meaning as that commonly understood by those of ordinary skill in the art to which the present disclosure pertains.

The technical solutions of the present disclosure are further described below through embodiments and in conjunction with the accompanying drawings. Unless otherwise stated, the methods and materials of the embodiments described below are all conventional products commercially available. It will be understood by those skilled in the art to which the present disclosure pertains that the methods and materials described below are merely exemplary, and should not be construed as limiting the scope of the present disclosure.

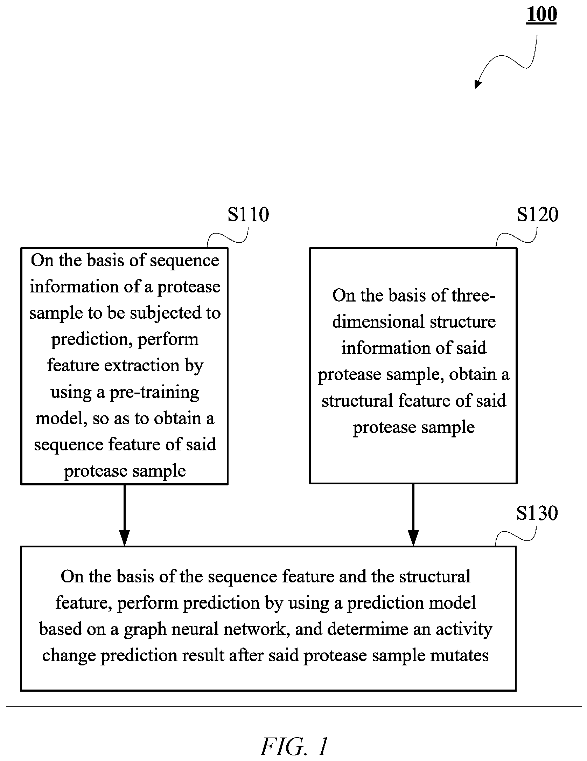

From the overall perspective of prediction methods, FIG. 1 shows a flow chart of a method for predicting the influence of a mutation on protease activity according to the embodiments of the present disclosure.

As shown in FIG. 1, the method 100 for predicting the influence of the mutation on protease activity according to the embodiments of the present disclosure starts at step S110. In this step, feature extraction is performed using a pre-trained model on the basis of sequence information of a protease sample to be predicted, so as to obtain a sequence feature of the protease sample to be predicted.

The sequence information of the protease sample to be predicted described here may include amino acid sequence information of the protease sample to be predicted before mutation and after mutation.

In another case, the sequence information of the protease sample to be predicted may also include amino acid sequence information of the protease sample to be predicted before mutation and specified mutation information. In this case, amino acid sequence information of the protease sample to be predicted after mutation can be obtained on the basis of the amino acid sequence information of the protease sample to be predicted before mutation and the specified mutation information. The specified mutation information may include a specified mutation site and/or a specified amino acid type after mutation. For example, in the case that the specified mutation information is a specified mutation site, the amino acid at this site can be altered to form the amino acid sequence information of the protease sample to be predicted after mutation. That is, in the case that the specified mutation information does not include a specified amino acid type after mutation, the amino acid sequence information after mutation may consist of 19 amino acid sequences, in which the amino acid at the mutation site is replaced with each of the other 19 types of amino acids. In the case that the specified mutation information further includes a specified amino acid type after mutation, the amino acid sequence information after mutation may be a single amino acid sequence. In another case that the specified mutation information only includes a specified amino acid type after mutation but not the mutation site, it is necessary to sequentially replace the amino acid at each site in the sequence before mutation with a specified amino acid type, thereby forming N sequences after mutation (where N is the number of sites in the sequence). In addition, in another extreme case that the specified mutation site includes multiple sites or even all sites, or the specified amino acid type after mutation includes multiple types or all the other 19 types, it is necessary to traverse all possible mutations to form up to 19*N sequences after mutation. The "amino acid type" described here refers to twenty distinct amino acids that constitute the primary building blocks of proteins in living organisms, including glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine, and histidine.

It should be understood by those skilled in the art that in subsequent steps, it is necessary to perform feature extraction separately on amino acid sequences before mutation and after mutation, which are then concatenated to form features related to the amino acid sequences. Therefore, regardless of the initially acquired protease sequence information, it is necessary to form amino acid sequences before mutation and after mutation when executing step S110.

Specifically, in step S110, feature extraction is performed separately on amino acid sequence information of a protease sample to be predicted before mutation and after mutation using a pre-trained model, so as to obtain sequence feature information before mutation and sequence feature information after mutation. Subsequently, still in step S110, the sequence feature information before mutation and the sequence feature information after mutation are concatenated, so as to obtain the sequence feature of the protease sample to be predicted.

In the research area of natural language processing, with the continuous enhancement of computer computing power, an increasing number of pre-trained models (PTMs) for general language representation have gradually emerged. This is of great help to a downstream preprocessing task, as it enables the learning of knowledge about language itself from massive unlabeled data, followed by fine-tuning on a small amount of labeled data, thereby allowing the downstream task to better learn both the inherent features of the language itself and the knowledge of a specific task. By training on a protein or nucleic acid sequence corpus, a pre-trained model applicable to the field of biology can be obtained. Existing models trained on the protein sequence corpus mainly include ESM-1b, UniRef, ProteinBert, TAPE, ProtGPT2, ProtTXL, ProtBert, ProtXLNet, ProtAlbert, ProtElectra, ProtT5-XL, and ProtT5-XXL. Although the pre-trained model can also be obtained by training on the protein sequence corpus by ourselves, the performance of such self-trained models generally fails to reach the level of the above models due to constraints in cost and time.

As described above, the pre-trained model is a natural language model in nature. In the field of biology, a pre-trained model that has been improved and optimised according to the characteristics and purposes of the field of biology is generally used. Such a pre-trained model has already learned knowledge related to the field of biology from a large amount of unlabeled sequence data in an existing protein corpus, and can be used to extract features related to mutation from the protein sequence information of training samples. Furthermore, by training on the basis of the extracted features related to mutation, model parameters can be optimised using only a small number of labeled training samples. For example, the pre-trained model described in the present disclosure may be implemented using one or a combination of more of the models mentioned above and listed again below: ESM-1b, UniRef, ProteinBert, TAPE, ProtGPT2, ProtTXL, ProtBert, ProtXLNet, ProtAlbert, ProtElectra, ProtT5-XL, and ProtT5-XXL. Preferably, the pre-trained model may be ProtT5-XL or ProtT5-XXL. More preferably, the pre-trained model is ProtT5-XL. That is, the pre-trained model used here is preferably a T5 (i.e., Transfer Text-to-Text Transformer) pre-trained model based on the latest natural language processing method.

The purpose of step S110 is to obtain the sequence feature of the protease sample to be predicted. The purpose of step S120, which will be described next, is to obtain a structural feature of the protease sample to be predicted.

In step S120, a structural feature of the protease sample to be predicted is obtained on the basis of three-dimensional structural information of the protease sample to be predicted.

In addition to obtaining related features through a pre-trained model, related features can also be obtained in combination with three-dimensional structural information of the protease for information supplementation. The three-dimensional structural information can be obtained from the PDB database (https://www.rcsb.org/) (see Berman, Helen M, et al., 2000, "The protein data bank", Nucleic Acids Research 28(1): 235-242, which is incorporated herein by reference in its entirety and made a part of the present disclosure) or by means of prediction software, such as the following (with reference descriptions or sources in parentheses):
AlphaFold2 (https://alphafold.ebi.ac.uk/);
I-TASSER (https://zhanggroup.org/I-TASSER/);
RoseTTAFold (see Minkyung Baek et al., "Accurate prediction of protein structures and interactions using a three-track neural network", Science 373, 871-876 (2021). DOI:10.1126/science.abj8754);
Modeller (https://salilab.org/modeller/); and
Swiss-model (https://swissmodel.expasy.org/).

The three-dimensional structural information of the protease that can be obtained usually includes a secondary structure, an interaction network, an amino acid residue distance, a physical environment, energy information derived from the three-dimensional structure, etc. Among them, the interaction network is one of the most commonly used features in structure-based prediction methods, as it contains contact information between all residue pairs.

According to preferred embodiments of the present disclosure, the three-dimensional structural information used is an interaction network. More specifically, prediction models based on Graph Neural Network (GNN) and various variants thereof are selected on the basis of the features of the interaction network.

It should be understood by those skilled in the art that the present disclosure does not limit the sequential order of step S110 and step S120. That is, the sequence feature and the structural feature can be obtained in any order, or simultaneously.

Finally, in step S130, prediction is performed using a graph neural network-based prediction model on the basis of the sequence feature obtained in step S110 and the structural feature obtained in step S120, so as to determine a prediction result for activity change of the protease sample to be predicted after mutation.

The graph neural network-based prediction model is actually a classifier.

In step S130, the sequence-based feature and the structure-based feature are input into the classifier to perform prediction for a specific task. Classifiers are generally classified into machine learning methods and deep learning methods. Commonly used machine learning methods include: linear regression algorithm, support vector machine (SVM) algorithm, K-nearest neighbors (KNN) algorithm, logistic regression (LR) algorithm, decision tree algorithm, K-means algorithm, random forest algorithm, naive Bayes algorithm, gradient boosting algorithm, and ensemble learning method. Deep learning methods generally include: convolutional neural network, recurrent neural network, recursive neural network, and long short-term memory (LSTM) network. A graph neural network (GNN) is a deep learning model based on graph-structured data, which is used to process structured data involving nodes and edges. A graph convolutional network (GCN) is a GNN model based on the convolutional neural network (CNN), which realises effective representation and learning of node features by aggregating information of neighboring nodes. A graph attention network (GAT) is a GNN model based on the attention mechanism, which realises adaptive aggregation of node features by learning the attention weights of neighboring nodes, and can better handle the relationships between different nodes. GraphSAGE (Graph Sampling and Aggregation, SageConv) is a GNN model based on sampling, which realises effective learning and representation of node features by sampling and aggregating neighboring nodes. GraphSAGE can handle large-scale graph data while ensuring the efficiency and accuracy of the model. A graph isomorphism network (GINConv) is a GNN model based on graph isomorphism, which realises effective learning and representation of node features by aggregating features of neighboring nodes and combining the aggregated result with the features of the node itself. A diffusion convolutional neural network (DCNN) is a GNN model based on a diffusion process, which realises effective learning and representation of node features by diffusing and aggregating node features. These methods all have their own advantages and application scopes, and appropriate methods can be selected on the basis of specific tasks and data characteristics.

In a preferred embodiment of the present disclosure, the graph neural network is a graph convolutional network or a graph attention network.

In addition, in a preferred embodiment of the present disclosure, in step S130, the sequence feature and the structural feature may first be input into the graph neural network, so as to obtain a probability feature related to activity change; then, the probability feature related to activity change is combined with supplementary features from other prediction methods, and the combined features are input into a decision tree model to finally obtain a classification of activity change. In a more preferred embodiment, the decision tree model used is a gradient boosting decision tree model. The decision tree model is implemented using a LightGBM framework. A more detailed description thereof will be provided in the subsequent description of the embodiments.

With regard to the method for constructing the prediction model, reference can be made to the detailed description below.

As described above, stability and catalytic activity are the two most important properties of proteases. Stability has been extensively studied, with dozens of AI-based methods developed to predict the influence of mutations on protease stability, demonstrating excellent performance. In contrast, catalytic activity prediction has stagnated due to limitations in datasets and methodologies. To date, only the SCANEER method can predict the influence of single-point mutations on protease activity. However, this method is based on co-evolutionary information, and therefore can only predict mutations that have occurred in the evolutionary process, posing significant limitations and thus failing to be widely applied.

The technique provided in the present disclosure differs from the above prior art in that the technique of the present disclosure introduces a graph neural network, particularly a graph convolutional network or a graph attention network, into the prediction of activity change of a protease after mutation, which is combined with a mature protein natural language model (pre-trained model) in the prior art to jointly contribute to the determination of a prediction result. In other words, the sequence feature (obtained from the primary sequence) and structural feature (obtained from the three-dimensional structure) of the protease sample to be predicted are combined to jointly serve as an input to a graph neural network-based prediction model, thereby outputting a prediction result related to activity change of the protease after mutation. The prediction result determined as such is superior to that determined by the methods in the prior art.

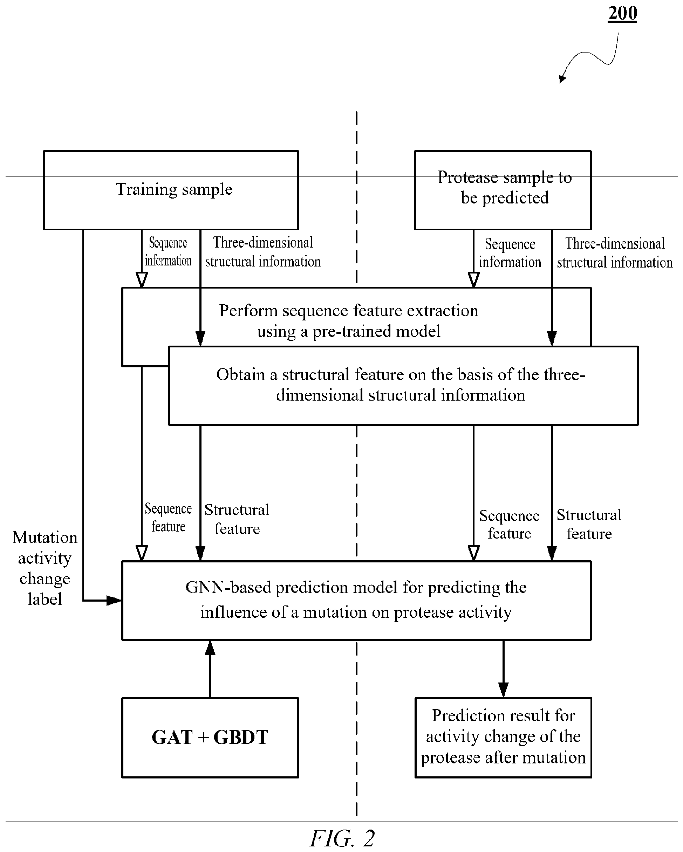

FIG. 2 shows a joint schematic diagram 200 of a method for predicting the influence of a mutation on protease activity according to the embodiments of the present disclosure, and a method for constructing a prediction model for predicting the influence of a mutation on protease activity according to the embodiments of the present disclosure. In the schematic diagram 200 as shown in FIG. 2, the left side of the dashed line shows a method for constructing a graph neural network-based prediction model, while the right side of the dashed line shows a prediction method using a graph neural network-based prediction model.

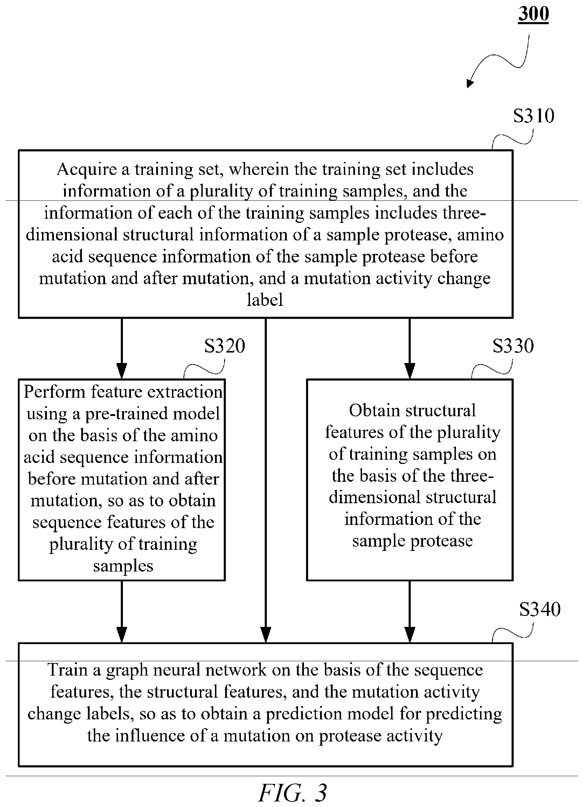

The flow on the right side of the dashed line in FIG. 2 is actually the method for predicting the influence of the mutation on protease activity according to the embodiments of the present disclosure as shown in FIG. 1. The flow on the left side of the dashed line in FIG. 2 will be further described in detail below (e.g., FIG. 3 and corresponding textual description thereof). As can be seen in FIG. 2, both the model construction and the actual prediction processes require the use of two feature extraction steps, namely: sequence feature extraction and structural feature extraction. For a training sample, three-dimensional structural information of a sample protease, and amino acid sequence information of the sample protease before mutation and after mutation are included, and a mutation activity change label is also included. As described below, the mutation activity change label may be protease activity strengthening or weakening (increased or decreased). Therefore, in the process of model construction, sequence information from training samples is used to extract sequence features. Structural features are extracted through three-dimensional structural information. The GNN-based prediction model, i.e., the classifier, is fully trained using the sequence feature and structural feature as input data, and the mutation activity change label from the training sample as output data, thereby obtaining the final GNN-based prediction model for predicting the influence of a mutation on protease activity. The constructed prediction model can then be put into practical use for predicting the mutation activity change of the protease, namely, the flow on the right side of the dashed line in FIG. 2. In addition, it should be noted that in FIG. 2, hollow arrows are used to indicate the signal transmission process for sequence information and sequence features; while solid arrows distinct from the hollow arrows are used to represent the signal transmission process for three-dimensional structural information and structural features. In addition, all other signal flows and control flows are also denoted using solid arrows.

The flow of the method for constructing the prediction model for predicting the influence of the mutation on protease activity according to the embodiments of the present disclosure will be described in detail below.

FIG. 3 is a flow chart of a method 300 for constructing a prediction model for predicting the influence of a mutation on protease activity as used in the present disclosure.

It is known to those skilled in the art that as shown in FIG. 3, in step S310, a training set should first be obtained, where the training set includes information of a plurality of training samples. Specifically, the information of each of the training samples includes three-dimensional structural information of the sample protease, amino acid sequence information of the sample protease before mutation and after mutation, and a mutation activity change label.

Artificial intelligence (AI) algorithms rely heavily on data. The quantity and quality of initial data determine the generalization performance of the trained model, which is most directly reflected in the prediction accuracy of the model. Insufficient sample size or poor dataset quality will lead to overfitting or underfitting in the model. To address this issue, the most effective method is to acquire more experimental data as the training set. The main source for acquiring experimental data currently is public databases. For the task of predicting the influence of a mutation on catalytic activity of a protease using a sequence-based method, the dataset size can be doubled by performing inversion processing on the data in the database. Inversion means that under the same environmental conditions, opposite mutations at the same position have opposite effects on activity. According to the above definition, merging the original data with the inverted data can double the original data volume. By using such an inversion method, the original dataset is not only expanded, but also balanced, which enhances the robustness of the final prediction model.

It should be noted that this method of expanding the dataset by inverting the original dataset is performed only for the training set.

In the specific embodiments below, the acquisition of the training dataset will be described in more detail.

The mutation activity change label can be used to reflect the trend of the influence of an amino acid mutation on catalytic activity of a protease. For example, the mutation activity change label may indicate that the catalytic activity is enhanced or weakened (increased or decreased). More specifically, reference can be made to the description of constructing the training dataset in specific embodiments below. It can be understood that using mutation activity change information as a label for a training sample enables the prediction model obtained through training to output a prediction result related to the influence of the amino acid mutation on catalytic activity of the protease. Preferably, the protease mutation mentioned in the present application may refer to a single-point mutation in the amino acid sequence of the protease.

In step S320, feature extraction is performed using a pre-trained model on the basis of amino acid sequence information of the sample protease before mutation and after mutation, so as to obtain sequence features of the plurality of training samples.

Specifically, in step S320, feature extraction is performed separately on the amino acid sequence information of the sample protease before mutation and after mutation using the pre-trained model, so as to obtain sequence feature information before mutation and sequence feature information after mutation. Subsequently, still in step S320, the sequence feature information before mutation and the sequence feature information after mutation are concatenated, so as to obtain the sequence features of the plurality of training samples.

As described above, the pre-trained model may be implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL. Preferably, the pre-trained model is ProtT5-XL or ProtT5-XXL, and more preferably, the pre-trained model is ProtT5-XL. That is, the pre-trained model used here is preferably a T5 (i.e., Transfer Text-to-Text Transformer) pre-trained model based on the latest natural language processing method.

The purpose of step S320 is to obtain the sequence feature of the training sample. The purpose of step S330, which will be described next, is to obtain the structural feature of the training sample.

In step S330, structural features of the plurality of training samples are obtained on the basis of the three-dimensional structural information of the sample protease.

As described above, the three-dimensional structural information of the sample protease can be obtained through at least one of the following databases or prediction software: PDB database, AlphaFold2, I-TASSER, RoseTTAFold, Modeller, Swiss-model, etc.

The three-dimensional structural information of the protease that can be obtained usually includes a secondary structure, an interaction network, an amino acid residue distance, a physical environment, energy information derived from the three-dimensional structure, etc. Among them, the interaction network is one of the most commonly used features in structure-based prediction methods, as it contains contact information between all residue pairs.

According to preferred embodiments of the present disclosure, the three-dimensional structural information used here is the interaction network. More specifically, a GNN-based prediction model is selected on the basis of the features of the interaction network.

It should be understood by those skilled in the art that the present disclosure does not limit the sequential order of step S320 and step S330. That is, the sequence feature and the structural feature can be obtained in any order, or simultaneously.

In step S340, a graph neural network is trained on the basis of the sequence features, the structural features, and the mutation activity change labels, so as to obtain the prediction model for predicting the influence of the mutation on protease activity.

As described above, the prediction model is actually a classifier.

In a preferred embodiment of the present disclosure, the graph neural network is a graph convolutional network (GCN) or a graph attention network (GAT).

With regard to the method for constructing the prediction model, reference can be made to the detailed description in the embodiments below.

In addition, it should be noted that: For sequence features, the pre-trained model may be transferred to a new task through transfer learning by inputting the processed protease activity change dataset into the pre-trained model. In transfer learning, there are two commonly used application methods: feature extraction and fine-tuning. In feature extraction, a simple classifier can be modified or added after pre-training a network structure. The pre-trained network for the original task is used as a feature extractor for another target task, where only parameters of the newly added classifier are re-learned, while parameters of the pre-trained network remain unmodified or frozen. In the preferred embodiments of the present disclosure, a transfer learning method based on feature extraction is used.

In the process of model training, sequence features and structural features are used as input data of the prediction model, and mutation activity change labels are used as output targets of the prediction model to fully train the prediction model. As the prediction model parameters are continuously optimised, the output data of the prediction model will be consistent with the prediction results represented by the mutation activity change labels.

In a preferred implementation of the present disclosure, step S340 may further include: inputting the sequence features and the structural features into the graph neural network, so as to obtain probability features related to activity change; then, combining the probability features related to activity change with supplementary features from other prediction methods, and inputting the combined features into a decision tree model to obtain a classification of activity change; and taking the mutation activity change labels as output targets, adjusting parameters of the prediction model.

More specifically, in some embodiments of the present disclosure, supervised training can be performed on the prediction model (classifier) on the basis of the sequence features, structural features, and mutation activity change labels of a plurality of training samples to obtain a trained classifier. Supervised training is a process of adjusting parameters of a classifier using a set of samples with known categories to achieve the required performance. In some embodiments, a loss function (such as a cross-entropy loss function) can be constructed. The loss function is used to reflect the difference between the prediction results of the catalytic activity change of the protease after mutation as predicted by the classifier and the actual mutation activity change labels of the training samples. Then, on the basis of this difference, the parameters of the classifier are adjusted to obtain the optimised model parameters of the classifier. When the loss function satisfies predetermined conditions, such as convergence of the loss function, the loss function value being less than a preset value, both the loss function values of the training set and the test set dropping to the minimum, or the loss function value of the training set dropping to the minimum, the training of the classifier is completed. In some embodiments, parameter search algorithms such as grid search and Bayesian search can be used to adjust the parameters of the classifier. In some embodiments, supervised training is a process of training the prediction model on the basis of mutation activity change labels.

It should be understood by those skilled in the art that the prediction model obtained, for example, by the method shown in FIG. 3 can be applied to the prediction method shown in FIG. 1.

Specific exemplary embodiments will be provided below.

### Embodiment methods and materials

### 1. Training dataset

To overcome the challenges caused by poor data quality, 5449 experimental samples containing activity change information were selected from the D3DistalMutation database published in 2021 (see Wang, Xiaoyu, et al., 2021, "D3DistalMutation: a database to explore the effect of distal mutations on enzyme activity", Journal of Chemical Information and Modeling 61(5): 2499-2508, which is incorporated herein by reference in its entirety and made a part of the present disclosure). These samples include 5279 activity-decreasing mutation samples and 220 activity-increasing mutation samples. The specific screening criteria are as follows:
(1) All multi-point mutation samples are removed and the training dataset is restricted to a single-point mutation dataset.
(2) Neutral mutations (mutations with no influence on protease activity) are removed, and mutations resulting in decreased or lost activity are uniformly defined as activity-decreasing mutations.
(3) Samples with different labels for the same mutation are removed. The reason for removing samples with different labels is as follows: The samples with different labels for the same mutation may result from different experimental conditions or experimental errors, making it difficult to define a label for such data. If such samples are not removed, they may introduce noise to the model and thus affect the accuracy.
(4) Data are inverted and merged.

Finally, the dataset used for training the model contains 10,898 samples.

### 2. Test dataset

The test data are derived from the BRENDA database (https://brenda-enzymes.org/news.php?) (see Chang, Antje, et al., 2021, "BRENDA, the ELIXIR core data resource in 2021: new developments and updates", Nucleic Acids Research 49(D1): D498-508, which is incorporated herein by reference in its entirety and made a part of the present disclosure.), literatures (see, for example, Amorosi, Clara J, et al., 2021,"Massively parallel characterization of CYP2C9 variant enzyme activity and abundance", The American Journal of Human Genetics 108(9): 1735-1751; Cheng, Ya-Shan, et al., 2015, "Improving the catalytic performance of a GH11 xylanase by rational protein engineering", Applied Microbiology and Biotechnology 99: 9503-9510; You, Chun, Hanying Yuan, Qiang Huang, and Hong Lu, 2010, "Substrate molecule enhances the thermostability of a mutant of a family 11 xylanase from Neocallimastix patriciarum", African Journal of Biotechnology 9(9), which are incorporated herein by reference in their entireties and made a part of the present disclosure), and patents (WO 2020234200 A1; EP 3854872 A1; WO 2018195850; and WO 2017109262 A1). The screening criteria are as follows:
(1) Non-single-point mutation samples are removed and the test dataset is restricted to a single-point mutation dataset.
(2) Neutral mutation samples are removed, and mutations resulting in decreased or lost activity are uniformly defined as activity-decreasing mutations.
(3) Samples with multiple labels are removed. Samples with multiple labels refer to those with different protease activities reported at the same mutation site. It is necessary to delete such mutation samples to ensure the accuracy of the data.
(4) Samples duplicated with those in the training dataset are removed.

Finally, the dataset used for testing the model performance contains 6103 samples, including 4950 activity-decreasing mutation samples and 1153 activity-increasing mutation samples.

### 2.1 S3459

Due to algorithmic limitations, SCANEER can only predict mutations that have occurred in the evolutionary process. Among the 6103 samples in the test set, 3459 samples can be predicted by SCANEER, of which 1797 are derived from DMS (Deep Mutational Scanning). DMS involves introducing random mutations into the gene encoding a protein, followed by evaluating the influence of these mutations on protein function through methods such as pressure screening or high-throughput sequencing. The remaining 1662 samples consist of non-DMS data.

### 2.2 S2644

Among all the test data, SCANEER cannot predict mutations for 2644 samples. Similar to Section 2.1, this part of the data is further divided into DMS data and non-DMS data, with sample sizes of 1492 and 1152, respectively.

### 3. Pre-trained model

The ProtT5-XL (ProtT5-XL-U50/ProtT5-XL-UniRef50) model used in this method was released by Ahmed Elnaggar et al. in 2021. This is a pre-trained protein model obtained by first training a T5 model on the BFD dataset and then fine-tuning the model on the UniRef50 dataset for optimisation. This model contains 24 attention layers, with each layer consisting of 32 self-attention heads and 1024 neurons. Reference can be made to Elnaggar, Ahmed, et al., 2021, "Prottrans: Toward understanding the language of life through self-supervised learning", IEEE Transactions on Pattern Analysis and Machine Intelligence 44(10): 7112-7127, which is incorporated herein by reference in its entirety and made a part of the present disclosure.

### 4. Construction of graph convolutional neural network model

This preferred embodiment is based on a variant of graph convolutional network (GCN): Graph Attention Network (GAT) (see Veličković, Petar, et al., 2017. "Graph Attention Networks". arXiv preprint arXiv:1710.10903*,* which is incorporated herein by reference in its entirety and made a part of the present disclosure).

### 4.1 Construction of GAT-1 model

### 4.1.1 Adjacency matrix

The three-dimensional structure of each protease was obtained using the PDB database, and prediction software such as AlphaFold2, I-TASSER, RoseTTAFold, Modeller, and Swiss-model. The distances between amino acid residue pairs in the three-dimensional structures of the proteases were calculated on the basis of the coordinates of the obtained three-dimensional structures of the proteases. In the case that the distance between alpha carbon atoms of two residue pairs was less than 10 angstroms, existence of an edge relationship between the nodes was indicated and recorded as 1, otherwise recorded as 0, with a self-connection recorded as 0, thereby forming the adjacency matrix of the entire protease.

In this embodiment, taking a mutation site as the centre, 10 amino acids were taken from each of the left side and the right side, resulting in a sequence of 10 consecutive amino acids as the residue sequence. A 21*21 adjacency matrix was then extracted from the adjacency matrix of the entire protease.

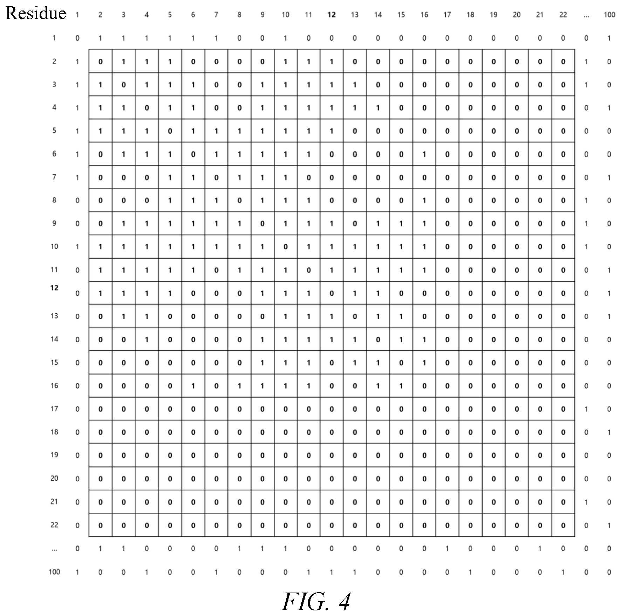

FIG. 4 is a schematic diagram of an adjacency matrix. An adjacency matrix corresponding to a protein sequence with 100 amino acids is shown in the figure. Assuming the mutation site is at residue 12, the boxed area represents the 21*21 adjacency matrix for this residue. The McBASCCovariance algorithm (see McLachlan, Andrew D., 1971, "Tests for comparing related amino-acid sequences. Cytochrome c and cytochrome c551", Journal of Molecular Biology 61(2): 409-424, which is incorporated herein by reference in its entirety and made a part of the present disclosure) was used to calculate pairwise co-evolution scores between the 21 corresponding amino acids in the adjacency matrix, forming a 21*21 co-evolution score matrix. This matrix was then multiplied by the adjacency matrix to obtain the updated 21*21 adjacency matrix.

### 4.1.2 Node feature matrix

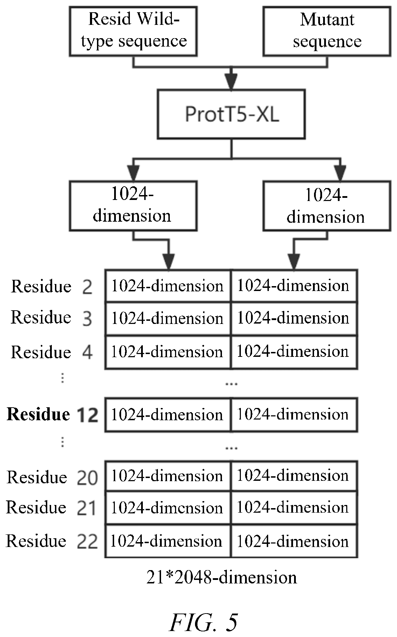

After a piece of protease sequence information was input into the pre-trained model, the model first encoded each amino acid in the protease sequence via an encoder. After encoding, each amino acid corresponded to a 1024-dimensional vector. Thus, for a protease sequence of length N, a vector of dimensions N* 1024 was output. The model consisted of 24 layers. To maximize the acquisition of mutation-related information, the last layer was selected as the output. The 1024-dimensional feature vectors corresponding to the mutation site of the wild type and the mutant were extracted separately, and the feature vectors of the wild type (i.e., before mutation) and the mutant (i.e., after mutation) were sequentially concatenated to form a 2048-dimensional vector. With a sliding window size of 21 and the mutation site as the centre, the 10 amino acid residues before and after the mutation site were concatenated. Finally, a 21*2048-dimensional feature was obtained as the node feature matrix. The structure is as shown in FIG. 5.

### 4.1.3 Model construction

The matrices generated in Sections 4.1.1 and 4.1.2 were input into the graph convolutional neural network as structural features and sequence features, respectively. Through matrix multiplication and aggregation operations, the three-layer graph network structure updated the node features into 32-dimensional, 64-dimensional, and 64-dimensional feature vectors, respectively. Four attention heads were used for each layer. The node features output by the three graph network layers were merged via a residual network structure into a 21*640-dimensional feature vector. Subsequently, an average pooling operation was used to compress the updated feature matrix into a 1*640-dimensional feature vector, which was used to represent the feature characterization of the sequence of 21 amino acid residues. The specific parameters are as follows: The output dimensions of the three graph convolution layers are 32, 64, and 64, respectively; the number of neurons of the fully connected layer is 64, 2; the optimiser used is Adam; the loss function is binary cross-entropy loss; 'relu' is used as the activation function; 'softmax' is used as the classification function of the output layer; the coefficient of the l2 regularization term is 0.05, and the dropout rate is 0.3; and the learning rate is 0.001.

The aggregation operation described here is implemented through an attention mechanism, where each node is assigned an attention weight distribution. By performing a weighted summation of the feature matrix and attention coefficients, the aggregated feature vector for the node may be obtained. Through such an aggregation operation, feature update and information propagation of each node can be performed using the feature information of neighboring nodes thereof.

The residual network described here is a deep convolutional neural network structure, which constructs skip connections between network layers by introducing residual connections. Specifically, the input to each network layer includes not only the output of the previous layer but also a directly connected skip connection. This skip connection directly adds the input to the output, forming a residual. The purpose of the residual network is to address the problems of gradient vanishing and difficulty in training deep networks.

The average pooling operation described here is a commonly used technique to reduce the feature dimensions output by convolutional layers, which is implemented by computing the average of the features output by the convolutional layers. The purpose of the average pooling operation is to effectively reduce the number of network parameters and prevent overfitting. It should be understood by those skilled in the art that the average pooling operation here can also be replaced by any pooling operation such as max pooling or K-max pooling.

### 4.2 Construction of GAT-2 model

The matrices generated in Sections 4.1.1 and 4.1.2 were directly input into the graph convolutional neural network. Through matrix multiplication and aggregation operations, each layer in the three-layer graph network structure updated the node features into 128-dimensional feature vectors. Four attention heads were used for each layer. The node features output by the three graph network layers were merged via a residual network structure into a 21*1536-dimensional feature vector. Subsequently, an average pooling operation was used to compress the updated feature matrix into a 1*1536-dimensional feature vector, which was used to represent the feature characterization of the sequence of 21 amino acid residues. The specific parameters are as follows: The output dimensions of the three graph convolution layers are 128, 128, and 128, respectively; the number of neurons of the fully connected layer is 64, 2; the optimiser used is Adam; the loss function is binary cross-entropy loss; 'relu' is used as the activation function; 'softmax' is used as the classification function of the output layer; the coefficient of the l2 regularization term is 0.01, and the dropout rate is 0.5; and the learning rate is 0.001.

### 4.3 Construction of GAT-3 model

### 4.3.1 First-order adjacency matrix

The adjacency matrix of the entire protease was first obtained according to Section 4.1.1. Then, all amino acids spatially having a distance of less than 10 angstroms from the residue at the mutation site were selected with the mutation site residue as the centre, and the adjacency matrix composed of these amino acids was calculated.

### 4.3.2 Node feature matrix

After a piece of protease sequence information was input into the pre-trained model, the model first encoded each amino acid in the protease sequence via an encoder. After encoding, each amino acid corresponded to a 1024-dimensional vector. Thus, for a protease sequence of length N, a vector of dimensions N*1024 was output. The model consisted of 24 layers. To maximize the acquisition of mutation-related information, the last layer was selected as the output in this embodiment. The 1024-dimensional feature vectors corresponding to the mutation site of the wild type and the mutant were extracted simultaneously, and the feature vectors of the wild type and the mutant were sequentially concatenated to form a 2048-dimensional vector. Finally, an N_{f}*2048 dimensional feature matrix was obtained as the node feature matrix, where N_{f} represents the number of corresponding amino acids obtained from Section 4.3.1, with their order maintained consistent. The number of amino acids obtained may vary among different proteases. Therefore, zero-padding was performed using the maximum N_{f} as a reference, ensuring that the feature matrices of all proteases had the same size.

### 4.3.3 Model construction

The matrices generated in Sections 4.3.1 and 4.3.2 were input into the graph convolutional neural network as structural features and sequence features, respectively. Through matrix multiplication and aggregation operations, the two-layer graph network structure updated the node features into 128-dimensional and 256-dimensional feature vectors, respectively. Four attention heads were used for each layer. The node features output by the two graph network layers were merged via a residual network structure into an N_{f}*1536-dimensional feature vector. Subsequently, an average pooling operation was used to compress the updated feature matrix into a 1*1536-dimensional feature vector, which was used to represent the feature characterization of the sequence of N_{f} amino acid residues. The specific parameters are as follows: The output dimensions of the two graph convolution layers are 128, 256, respectively; the number of neurons of the fully connected layer is 128, 2; the optimiser used is Adam; the loss function is binary cross-entropy loss; 'relu' is used as the activation function; 'softmax' is used as the classification function of the output layer; the coefficient of the l2 regularization term is 0.05, and the dropout rate is 0.3; and the learning rate is 0.001.

### 5. Construction of LightGBM

Gradient boosting decision tree (GBDT) (see Friedman, Jerome H., 2001, "Greedy function approximation: a gradient boosting machine". Annals of Statistics, 1189-1232, which is incorporated herein by reference in its entirety and made a part of the present disclosure) is a commonly used model in machine learning. The core idea thereof is to iteratively train weak classifiers (decision trees) to obtain an optimal model. In contrast, LightGBM (Light Gradient Boosting Machine, or simply LGBM) is a framework for implementing the GBDT algorithm (see Ke, Guolin, et al., 2017, "LightGBM: A Highly Efficient Gradient Boosting Decision Tree", Advances in Neural Information Processing Systems 30, which is incorporated herein by reference in its entirety and made a part of the present disclosure). The version of LightGBM used in the method of the present disclosure is 3.3.3.

### 5.1 Construction of LGBM-1 model

### 5.1.1 Feature extraction

Probability values (representing the probability of increased protease activity) were obtained as a one-dimensional feature from the GAT-1 model constructed in Section 4.1. Eleven additional dimensions of features were supplemented through the feature extraction described on the basis of PremPS (see Chen, Yuting, et al., 2020, "PremPS: Predicting the impact of missenses mutations on protein stability", PLoS Computational Biology 16(12): e1008543, which is incorporated herein by reference in its entirety and made a part of the present disclosure) and SCANEER (as described above) methods. A total of 12-dimensional features were obtained after concatenation. Among them, 10 dimensions were supplemented by PremPS and 1 dimension by SCANEER.

### 5.1.2 Model construction

The obtained 12-dimensional features were input into the LightGBM framework for training, with the learning rate set to 0.05, extra_trees set to True, and other parameters using default values.

### 5.2 Construction of LGBM-2 model

### 5.2.1 Feature extraction

Similar to Section 5.1.1, probability values (representing the probability of increased protease activity) were obtained as two-dimensional features from the GAT-2 and GAT-3 models constructed in Sections 4.2 and 4.3, respectively. Eleven additional dimensions of features were supplemented through the feature extraction described on the basis of PremPS and SCANEER methods. A total of 13-dimensional features were obtained after concatenation.

### 5.2.2 Model construction

The obtained 13-dimensional features were input into the LightGBM framework for training, with the learning rate set to 0.05, extra_trees set to True, and other parameters using default values.

A summary is provided below.

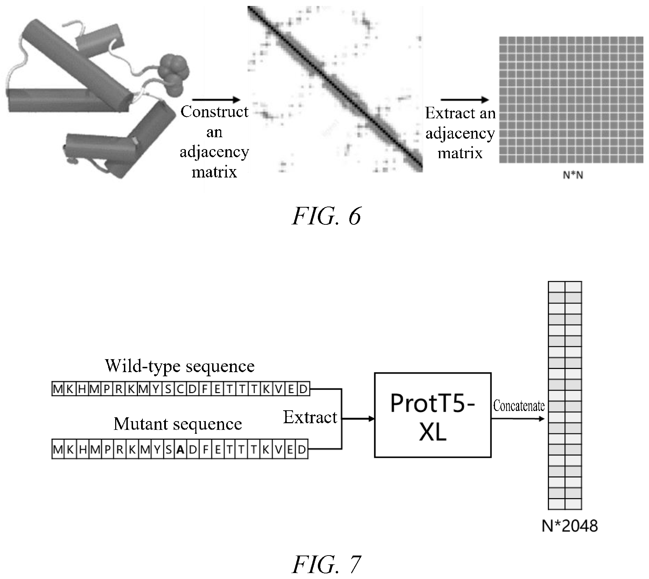

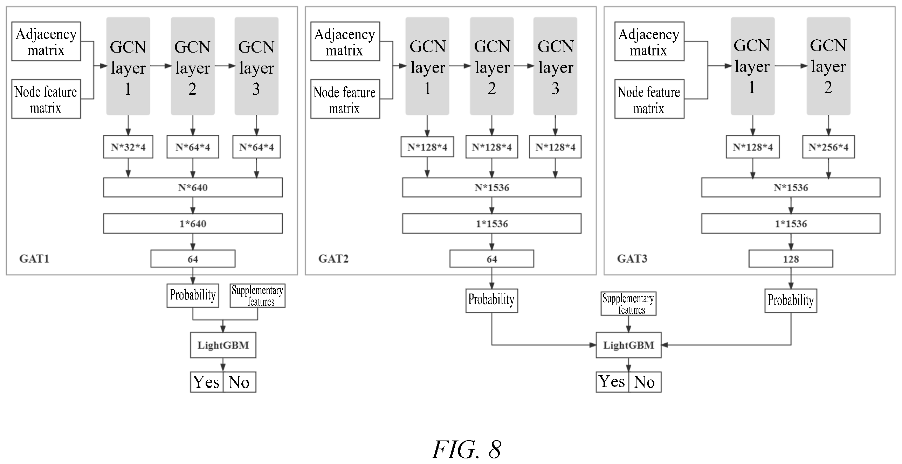

FIG 6 shows a schematic diagram illustrating the structure-based construction of an adjacency matrix. FIG 7 shows a schematic diagram illustrating the sequence-based construction of a node feature matrix. FIG. 8 shows a schematic diagram of the framework of the prediction model.

On the basis of the above embodiments, it can be seen that in the process of constructing the prediction model, the three-dimensional structural information of the sample protease can be acquired by: acquiring coordinates of a three-dimensional structure of the sample protease; calculating distances between amino acid residue pairs in the three-dimensional structure of the sample protease on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire sample protease. Said obtaining the structural features of the plurality of training samples on the basis of the three-dimensional structural information of the sample protease, may include: taking a mutation site as the centre, extracting an adjacency matrix of a specified size from the adjacency matrix of the entire sample protease, which is used as the structural feature of the sample protease. Said training the graph neural network on the basis of the sequence features, the structural features, and the mutation activity change labels, so as to obtain the prediction model for predicting the influence of the mutation on protease activity, may further include: inputting the sequence features and the structural features into the graph neural network, so as to obtain probability features related to activity change; combining the probability features related to activity change with supplementary features from other prediction methods, and inputting the combined features into a decision tree model to obtain a classification of activity change; and taking the mutation activity change labels as output targets, adjusting parameters of the prediction model. Said inputting the sequence features and the structural features into the graph neural network, so as to obtain probability features related to activity change, may further include: inputting the sequence features and the structural features into the graph neural network; updating the features through matrix multiplication and aggregation operations using several layers of graph network structures, and then outputting the updated features; merging the features output by the several layers of graph network structures, respectively; compressing the features using a pooling operation; and performing activity change classification on the features on the basis of a classification function of an output layer, and outputting the probability features related to activity change. The pooling operation described here may be average pooling, max pooling, or K-max pooling.

Correspondingly, in the process of applying the prediction model to the prediction of activity change of the protease after mutation, the three-dimensional structural information of the protease sample to be predicted can be acquired by: acquiring coordinates of a three-dimensional structure of the protease sample to be predicted; calculating distances between amino acid residue pairs in the three-dimensional structure of the protease on the basis of the acquired coordinates of the three-dimensional structure; and when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire protease sample to be predicted. Said obtaining the structural feature of the protease sample to be predicted on the basis of three-dimensional structural information of the protease sample to be predicted, includes: taking a mutation site as the centre, extracting an adjacency matrix of a specified size from the adjacency matrix of the entire protease sample to be predicted, which is used as the structural feature of the protease sample to be predicted. Said performing prediction using the graph neural network-based prediction model on the basis of the sequence feature and the structural feature, so as to determine the prediction result for activity change of the protease sample to be predicted after mutation, may further include: inputting the sequence feature and the structural feature into the graph neural network, so as to obtain a probability feature related to activity change; and combining the probability feature related to activity change with supplementary features from other prediction methods, and inputting the combined features into a decision tree model to obtain a classification of activity change. More specifically, said inputting the sequence feature and the structural feature into the graph neural network, so as to obtain the probability feature related to activity change, may include: inputting the sequence feature and the structural feature into the graph neural network; updating the features through matrix multiplication and aggregation operations using several layers of graph network structures, and then outputting the updated features; merging the features output by the several layers of graph network structures, respectively; compressing the features using a pooling operation; and performing activity change classification on the features on the basis of a classification function of an output layer, and outputting the probability feature related to activity change. Similarly, the pooling operation described here may be average pooling, max pooling, or K-max pooling.

### 6. Performance indicators

In this embodiment, evaluation was performed using the following indicators: area under the curve (AUC), precision, and recall. The specific definitions are as follows:
True Positive (TP)
False Positive (FP)
True Negative (TN)
False Negative (FN)

Among them, TP (True Positive) and TN (True Negative) represent the numbers of correctly classified positive samples and negative samples, respectively, FP (False Positive) and FN (False Negative) represent the numbers of incorrectly classified positive samples and negative samples, respectively, and P and N represent the numbers of positive samples and negative samples, respectively.

AUC is a popular parameter-independent metric used to describe a binary classifier. AUC refers to the area under the receiver operating characteristic curve (ROC), where a larger value indicates better performance of the classifier, with the maximum value being 1.

Precision refers to the proportion of correctly predicted results among all results predicted as positive samples. The calculation formula for precision is as follows: precision = TP/(TP + FP), where TP (True Positive) refers to correctly classified positive samples, and FP (False Positive) refers to incorrectly classified positive samples.

Recall refers to the proportion of the number of correctly predicted positive samples to the total number of positive samples. The calculation formula for recall is as follows: recall = TP/(TP + FN), where FN (False Negative) refers to incorrectly classified negative samples.

### Result evaluation

### 1. Model performance

By means of training the model on a self-designed balanced dataset, which includes 10,898 single-point mutations of 1303 proteases from the D3DistalMutation database, the influence of these mutations on enzyme activity was evaluated. During five-fold cross-validation, the performance of the model on the test data is shown in Table 1 below.

**Table 1: Performance indicators of the prediction model of the present disclosure on the training set using five-fold cross-validation**

| Evaluation indicator | Precision | Recall | AUC |
|---|---|---|---|
| LGBM-1 | 0.96 | 0.93 | 0.97 |
| LGBM-2 | 0.95 | 0.96 | 0.99 |

### 2. Comparison with other methods

To evaluate the differences between the method of the present disclosure and the SCANEER method, the model of the present disclosure was compared with SCANEER, the only existing prediction method. SCANEER evaluates the co-evolutionary relationships between amino acid pairs in multiple sequence alignments (MSAs). Amino acid pairs, i.e., amino acid substitutions, that are not observed or rarely observed in MSAs cannot be predicted, ultimately resulting in predictable mutations for each protease accounting for only 47% of all possible mutations (the authors of SCANEER hypothesized that amino acid substitutions that are not observed or rarely observed in MSA would lead to loss of protease function and have been eliminated during evolution. Therefore, the authors excluded positions with a gap percentage exceeding 20% or those not belonging to the top N coevolutionary amino acid pairs. That is, in MSA, for amino acids at positions with a gap percentage exceeding 20%, fully conserved positions, and positions not belonging to the top N coevolutionary amino acid pairs (where N = protease length × 2), the effects of mutations cannot be calculated), which greatly limits the application scope of this method. Therefore, among the 6103 samples in the experimental test set, SCANEER could predict 3459 samples, including 2855 activity-decreasing mutation samples and 604 activity-increasing mutation samples. Among these 3459 samples, the sample sizes of DMS data and non-DMS data were 1797 and 1662, respectively. The comparison results are as shown in Table 2 below.

**Table 2: Performance comparison between the model of the present disclosure and the SCANEER method on comparable experimental test sets**

| Test set | Method | Evaluation indicator | | |
|---|---|---|---|---|
| | | Precision | Recall | AUC |
| S3459 | LGBM-1 | 0.77 | 0.12 | 0.69 |
| | LGBM-2 | 0.74 | 0.15 | 0.68 |
| | SCANEER | 0.75 | 0.10 | 0.68 |
| S1797 | LGBM-1 | 0.67 | 0.05 | 0.73 |
| | LGBM-2 | 0.70 | 0.09 | 0.75 |
| | SCANEER | 0.50 | 0.05 | 0.73 |
| S1662 | LGBM-1 | 0.78 | 0.13 | 0.68 |
| | LGBM-2 | 0.74 | 0.16 | 0.68 |
| | SCANEER | 0.78 | 0.11 | 0.66 |

As can be seen from the above table, on S3459 and S1662, the method of the present disclosure was slightly superior to or basically comparable with SCANEER in terms of precision and recall, while on S1797, the method had a significant advantage in precision.

Dataset S2644 consists of data that cannot be predicted by SCANEER, including 2095 activity-decreasing mutation samples and 549 activity-increasing mutation samples. For this part of the data, the sample sizes of DMS data and non-DMS data were 1492 and 1152, respectively. The performance of the method of the present disclosure on this part of the dataset is shown in Table 3 below.

**Table 3: Performance of the model of the present disclosure on experimental test data that cannot be predicted by SCANEER**

| Data | Method | Evaluation indicator | | |
|---|---|---|---|---|
| | | Precision | Recall | AUC |
| S2644 | LGBM-1 | 0.69 | 0.10 | 0.71 |
| | LGBM-2 | 0.73 | 0.15 | 0.68 |
| S1492 | LGBM-1 | 0.81 | 0.13 | 0.81 |
| | LGBM-2 | 0.78 | 0.19 | 0.78 |
| S1152 | LGBM-1 | 0.62 | 0.08 | 0.72 |
| | LGBM-2 | 0.70 | 0.14 | 0.72 |

The results showed that the method of the present disclosure still achieved good performance on the experimental test data that cannot be predicted by SCANEER, indicating the advantages exhibited by the method of the present disclosure.

### Prediction process

Here, two cases were taken as examples to describe the process of predicting activity change of the protease using the prediction model.

One case was that the input protease sequence information included a single protease sequence and mutation site information. First, on the basis of the input single protease sequence and mutation site information, protease mutation site preprocessing was performed. Specifically, the input protease sequence was recorded as an original sequence, and a sequence after mutation was obtained on the basis of the mutation site information. Then, amino acid embedding layer extraction was performed, which was the sequence information extraction described above. That is, the original sequence was input into a natural language processing T5 pre-trained model to read embedding features; the protease sequence after mutation was input into a natural language processing T5 pre-trained model to read embedding features; amino acid embedding features were extracted on the basis of the mutation site, and the features before and after mutation were concatenated as an input for the subsequent model. Next, a graph network was constructed, which required a protease embedding layer feature network and a protease residue interaction feature network. The above feature networks were applied to a GAT-based prediction model to predict a probability of activity change of the protease before and after amino acid mutation. Next, on the basis of the probability of activity change of the protease, the activity change of the protease before and after amino acid mutation was predicted using LightGBM. Finally, the prediction results were output and displayed. For the case exemplified here, the prediction results for single-point mutations could be output.

Another case was that the input protease sequence information only included a single protease sequence. First, on the basis of the input single protease sequence, protease mutation site preprocessing was performed. Specifically, the input protease sequence was recorded as an original sequence, and the amino acid at each site in the protease sequence was sequentially mutated to each of the other 19 types of amino acids, with each resulting sequence recorded as a sequence after mutation. Then, amino acid embedding layer extraction was performed, which was the sequence information extraction described above. That is, the original sequence was input into a natural language processing T5 pre-trained model to read embedding features; the protease sequence after mutation was input into a natural language processing T5 pre-trained model to read embedding features; amino acid embedding features were extracted on the basis of the mutation site, and the features before and after mutation were concatenated as an input for the subsequent model. Next, a graph neural network was constructed, which required a protease embedding layer feature network and a protease residue interaction feature network. The above feature networks were applied to a GAT-based prediction model to predict a probability of activity change of the protease before and after amino acid mutation. Next, on the basis of the probability of activity change of the protease, the activity change of the protease before and after amino acid mutation was predicted using LightGBM. Finally, the prediction results were output and displayed. For the scenario exemplified here, the final prediction results could be output as a table of probabilities for all activity-increasing mutations and/or a ranking of the possibilities of all activity-increasing mutations.

### Conclusion

Protease mutants with higher activity are essential for the academic and industrial sectors to explore additional applications of these proteases. The present disclosure proposes a new method for identifying the influence of amino acid mutations on protease activity. The method of the present disclosure is based on an advanced natural language processing model and a graph neural network model. By means of integrating sequence and three-dimensional structural information, the method can be applied to various biological fields and exhibits performance superior to other methods, demonstrating broad application prospects of the method in protease engineering.

Discussion on the advantages of the method of the present disclosure over the prior art is provided below. As can be seen from the above description:
1. In summary, in the method of the present disclosure, a prediction model is constructed through AI training on the basis of the tertiary structure and amino acid sequence information of proteases.
2. The prediction model constructed in the present disclosure can be applied to predict the influence of a mutation in protease on protease activity.
3. After the training dataset used in the method of the present disclosure is reversed, the original dataset is not only expanded but also balanced, thereby enhancing the robustness of the final prediction model.

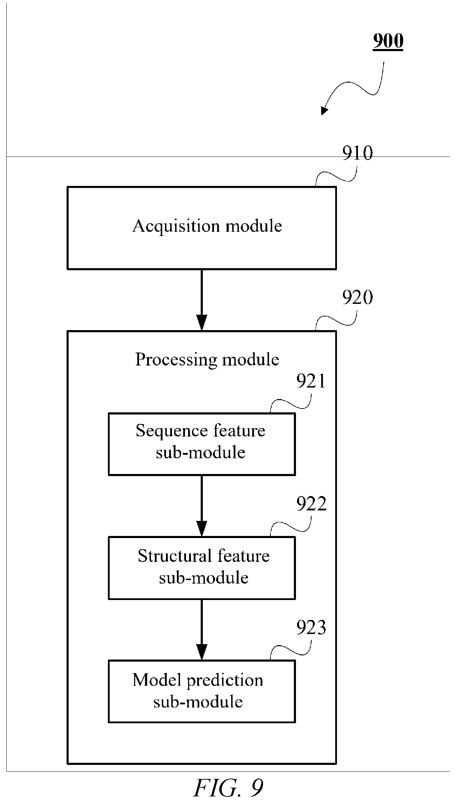

It should be understood by those skilled in the art that on the basis of the method for predicting the influence of the mutation on protease activity of the present disclosure, a system for predicting the influence of a mutation on protease activity can be developed. FIG. 9 shows a schematic block diagram of a system for predicting the influence of a mutation on protease activity according to the present disclosure. Specifically, a system 900 for predicting the influence of a mutation on protease activity includes an acquisition module 910 and a processing module 920.

The acquisition module 910 is configured to acquire sequence information and three-dimensional structural information of a protease sample to be predicted.

The processing module 920 is configured to obtain a prediction result for activity change of the protease sample to be predicted after mutation by inputting the sequence information and the three-dimensional structural information of the protease sample to be predicted. As shown in FIG. 9, the processing module 920 may further include: a sequence feature sub-module 921, configured to perform feature extraction using a pre-trained model on the basis of the sequence information of the protease sample to be predicted, so as to obtain a sequence feature of the protease sample to be predicted; a structural feature sub-module 922, configured to obtain a structural feature of the protease sample to be predicted on the basis of the three-dimensional structural information of the protease sample to be predicted; and a model prediction sub-module 923, configured to perform prediction using a graph neural network-based prediction model on the basis of the sequence feature and the structural feature, so as to determine a prediction result for activity change of the protease sample to be predicted after mutation.

It should be understood by those skilled in the art that the system for predicting the influence of the mutation on protease activity described above can be implemented by a computer. For example, the following operations can be implemented by executing a computer program: acquiring sequence information and three-dimensional structural information of a protease sample to be predicted; obtaining a prediction result for activity change of the protease sample to be predicted after mutation by inputting the sequence information and the three-dimensional structural information of the protease sample to be predicted, where the operation further includes the following sub-operations: performing feature extraction using a pre-trained model on the basis of sequence information of a protease sample to be predicted, so as to obtain a sequence feature of the protease sample to be predicted; obtaining a structural feature of the protease sample to be predicted on the basis of three-dimensional structural information of the protease sample to be predicted; and performing prediction using a graph neural network-based prediction model on the basis of the sequence feature and the structural feature, so as to determine a prediction result for activity change of the protease sample to be predicted after mutation.

That is, although in the system for predicting the influence of the mutation on protease activity according to the present disclosure, functions corresponding to individual operations of the prediction method are described as modules or sub-modules, it should be understood by those skilled in the art that such modules or sub-modules may be circuit components or other physical assemblies, or they may not be composed of circuit components or other physical assemblies, but rather functional modules constructed through computer programs.

In addition, it should be recognized by those skilled in the art that the method of the present disclosure can be implemented as a computer program. As described above in conjunction with the accompanying drawings, the method of the above embodiment is executed by one or more programs, and instructions in the programs cause a computer or processor to execute the algorithm described in conjunction with the accompanying drawings. These programs can be stored using various types of non-transitory computer-readable media and provided to a computer or processor. Non-transitory computer-readable media include various types of tangible storage media. Examples of non-transitory computer-readable media include magnetic recording media (such as floppy disks, magnetic tapes, and hard disk drives), magneto-optical recording media (such as magneto-optical disks), CD-ROMs (Compact Disc Read-Only Memories), CD-R, CD-R/W, and semiconductor memories (such as ROMs (Read-Only Memories), PROMs (Programmable ROMs), EPROMs (Erasable Programmable ROMs), flash ROMs, and RAMs (Random Access Memory)). Further, these programs can be provided to a computer using various types of transitory computer-readable media. Examples of transitory computer-readable media include electrical signals, optical signals, and electromagnetic waves. Transitory computer-readable media can be configured to provide programs to a computer via wired communication paths such as electric wires and optical fibers, or wireless communication paths.

For example, according to an embodiment of the present disclosure, a non-transitory computer-readable storage medium may be provided for storing a computer program, where the computer program includes instructions which, when executed by a processor of an electronic device, cause the electronic device to perform the method for predicting the influence of the mutation on protease activity described above.

In addition, according to the present disclosure, a computer system may further be provided. The computer system includes: a processor; a memory; and a computer program. The computer program is stored in the memory and configured to be executed by the processor. The computer program includes instructions for performing the method for predicting the influence of the mutation on protease activity as described above.

In another aspect, according to an embodiment of the present disclosure, a non-transitory computer-readable storage medium may be provided for storing a computer program, where the computer program includes instructions which, when executed by a processor of an electronic device, cause the electronic device to perform the method for constructing the prediction model for predicting the influence of the mutation on protease activity described above.

In addition, according to the present disclosure, a computer system may further be provided. The computer system includes: a processor; a memory; and a computer program. The computer program is stored in the memory and configured to be executed by the processor. The computer program includes instructions for performing the method for constructing the prediction model for predicting the influence of the mutation on protease activity described above.

The implementations of the present disclosure are not limited to those described in the above embodiments. Without departing from the spirit and scope of the present disclosure, those of ordinary skill in the art can make various changes and improvements to the present disclosure in form and detail, and all such changes and improvements are deemed to fall within the scope of protection of the present disclosure.

## Claims

1. A method for predicting the influence of a mutation on protease activity, **characterized in that** the method comprises:
performing feature extraction using a pre-trained model on the basis of sequence information of a protease sample to be predicted, so as to obtain a sequence feature of the protease sample to be predicted;
obtaining a structural feature of the protease sample to be predicted on the basis of three-dimensional structural information of the protease sample to be predicted; and
performing prediction using a graph neural network-based prediction model on the basis of the sequence feature and the structural feature, so as to determine a prediction result for activity change of the protease sample to be predicted after mutation.

2. The method according to claim 1, **characterized in that** the sequence information of the protease sample to be predicted comprises amino acid sequence information of the protease sample to be predicted before mutation and after mutation.

3. The method according to claim 1, **characterized in that** the sequence information of the protease sample to be predicted comprises amino acid sequence information of the protease sample to be predicted before mutation and specified mutation information.

4. The method according to claim 3, **characterized in that** the method further comprises: obtaining amino acid sequence information of the protease sample to be predicted after mutation on the basis of the amino acid sequence information of the protease sample to be predicted before mutation and the specified mutation information.

5. The method according to claim 3 or 4, **characterized in that** the specified mutation information comprises a specified mutation site and/or a specified amino acid type after mutation.

6. The method according to claim 2 or 4, **characterized in that** said performing feature extraction using the pre-trained model on the basis of the sequence information of the protease sample to be predicted, so as to obtain the sequence feature of the protease sample to be predicted, further comprises:
performing feature extraction separately on the amino acid sequence information of the protease sample to be predicted before mutation and after mutation using the pre-trained model, so as to obtain sequence feature information before mutation and sequence feature information after mutation; and
concatenating the sequence feature information before mutation and the sequence feature information after mutation, so as to obtain the sequence feature of the protease sample to be predicted.

7. The method according to claim 1, **characterized in that** the pre-trained model is implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

8. The method according to claim 1, **characterized in that** the three-dimensional structural information of the protease sample to be predicted comprises three-dimensional structural information of the protease sample to be predicted acquired through at least one of the following databases or prediction software: PDB database, AlphaFold2, I-TASSER, RoseTTAFold, Modeller, and Swiss-model.

9. The method according to claim 1, **characterized in that** the three-dimensional structural information of the protease sample to be predicted comprises an interaction network, a secondary structure, an amino acid residue distance, or a physical environment; preferably, the three-dimensional structural information of the protease sample to be predicted comprises an interaction network.

10. The method according to claim 8 or 9, **characterized in that** the three-dimensional structural information of the protease sample to be predicted is further acquired by:
acquiring coordinates of a three-dimensional structure of the protease sample to be predicted;
calculating distances between amino acid residue pairs in the three-dimensional structure of the protease on the basis of the acquired coordinates of the three-dimensional structure; and
when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire protease sample to be predicted.

11. The method according to claim 10, **characterized in that** said obtaining the structural feature of the protease sample to be predicted on the basis of three-dimensional structural information of the protease sample to be predicted, comprises:
taking a mutation site as the center, extracting an adjacency matrix of a specified size from the adjacency matrix of the entire protease sample to be predicted, which is used as the structural feature of the protease sample to be predicted.

12. The method according to claim 1, **characterized in that** said performing prediction using the graph neural network-based prediction model on the basis of the sequence feature and the structural feature, so as to determine the prediction result for activity change of the protease sample to be predicted after mutation, further comprises:
inputting the sequence feature and the structural feature into the graph neural network, so as to obtain a probability feature related to activity change; and
combining the probability feature related to activity change with supplementary features from other prediction methods, and inputting the combined features into a decision tree model to obtain a classification of activity change.

13. The method according to claim 12, **characterized in that** said inputting the sequence feature and the structural feature into the graph neural network, so as to obtain the probability feature related to activity change, comprises:
inputting the sequence feature and the structural feature into the graph neural network;
updating the features through matrix multiplication and aggregation operations using several layers of graph network structures, and then outputting the updated features;
merging the features output by the several layers of graph network structures, respectively;
compressing the features using a pooling operation; and
performing activity change classification on the features on the basis of a classification function of an output layer, and outputting the probability feature related to activity change.

14. The method according to claim 1, **characterized in that** the graph neural network-based prediction model is constructed through the steps of:
acquiring a training set, wherein the training set comprises information of a plurality of training samples, and the information of each of the training samples comprises three-dimensional structural information of a sample protease, amino acid sequence information of the sample protease before mutation and after mutation, and a mutation activity change label;
performing feature extraction using a pre-trained model on the basis of the amino acid sequence information of the sample protease before mutation and after mutation, so as to obtain sequence features of the plurality of training samples;
obtaining structural features of the plurality of training samples on the basis of the three-dimensional structural information of the sample protease; and
training a graph neural network on the basis of the sequence features, the structural features, and the mutation activity change labels, so as to obtain the graph neural network-based prediction model.

15. The method according to claim 14, **characterized in that** the graph neural network-based prediction model is a classifier.

16. The method according to claim 14, **characterized in that** the graph neural network is a graph convolutional network or a graph attention network.

17. The method according to claim 14, **characterized in that** said training the graph neural network on the basis of the sequence features, the structural features, and the mutation activity change labels, so as to obtain the graph neural network-based prediction model, further comprises:
inputting the sequence features and the structural features into the graph neural network, so as to obtain probability features related to activity change;
combining the probability features related to activity change with supplementary features from other prediction methods, and inputting the combined features into a decision tree model to obtain a classification of activity change; and
taking the mutation activity change labels as output targets, adjusting parameters of the prediction model.

18. The method according to claim 12 or 17, **characterized in that** the decision tree model is a gradient boosting decision tree (GBDT) model.

19. The method according to claim 18, **characterized in that** the GBDT model is implemented using a LightGBM framework.

20. The method according to claim 14, **characterized in that** the step of acquiring the training set comprises:
acquiring experimental data from a public database as a training set; and
doubling the size of the training set by performing inversion processing on the experimental data from the public database.

21. A method for constructing a prediction model for predicting the influence of a mutation on protease activity, **characterized in that** the method comprises:
acquiring a training set, wherein the training set comprises information of a plurality of training samples, and the information of each of the training samples comprises three-dimensional structural information of a sample protease, amino acid sequence information of the sample protease before mutation and after mutation, and a mutation activity change label;
performing feature extraction using a pre-trained model on the basis of the amino acid sequence information of the sample protease before mutation and after mutation, so as to obtain sequence features of the plurality of training samples;
obtaining structural features of the plurality of training samples on the basis of the three-dimensional structural information of the sample protease; and
training a graph neural network on the basis of the sequence features, the structural features, and the mutation activity change labels, so as to obtain the prediction model for predicting the influence of the mutation on protease activity.

22. The method according to claim 21, **characterized in that** said performing feature extraction using the pre-trained model on the basis of the amino acid sequence information of the sample protease before mutation and after mutation, so as to obtain the sequence features of the plurality of training samples, comprises:
performing feature extraction separately on the amino acid sequence information of the sample protease before mutation and after mutation using the pre-trained model, so as to obtain sequence feature information before mutation and sequence feature information after mutation; and
concatenating the sequence feature information before mutation and the sequence feature information after mutation, so as to obtain the sequence features of the plurality of training samples.

23. The method according to claim 21 or 22, **characterized in that** the pre-trained model is implemented using one or a combination of more of the following models: ESM-1b, UniRef, ProteinBert, TAPE, ProtGPT2, ProtTXL, ProtBert, ProtXLNet, ProtAlbert, ProtElectra, ProtT5-XL, and ProtT5-XXL; preferably, the pre-trained model is ProtT5-XL or ProtT5-XXL; and more preferably, the pre-trained model is ProtT5-XL.

24. The method according to claim 21, **characterized in that** the three-dimensional structural information of the sample protease comprises three-dimensional structural information of the sample protease acquired through at least one of the following databases or prediction software: PDB database, AlphaFold2, I-TASSER, RoseTTAFold, Modeller, and Swiss-model.

25. The method according to claim 21, **characterized in that** the three-dimensional structural information of the sample protease comprises an interaction network, a secondary structure, an amino acid residue distance, or a physical environment; preferably, the three-dimensional structural information of the sample protease comprises an interaction network.

26. The method according to claim 24 or 25, **characterized in that** the three-dimensional structural information of the sample protease is further acquired by:
acquiring coordinates of a three-dimensional structure of the sample protease;
calculating distances between amino acid residue pairs in the three-dimensional structure of the sample protease on the basis of the acquired coordinates of the three-dimensional structure; and
when the distance between alpha carbon atoms of two residue pairs is less than 10 angstroms, indicating existence of an edge relationship between nodes, recorded as 1, otherwise recorded as 0, with a self-connection recorded as 0, thereby obtaining an adjacency matrix of an entire sample protease.

27. The method according to claim 26, **characterized in that** said obtaining the structural features of the plurality of training samples on the basis of the three-dimensional structural information of the sample protease, comprises:
taking a mutation site as the centre, extracting an adjacency matrix of a specified size from the adjacency matrix of the entire sample protease, which is used as the structural feature of the sample protease.

28. The method according to claim 21, **characterized in that** said training the graph neural network on the basis of the sequence features, the structural features, and the mutation activity change labels, so as to obtain the prediction model for predicting the influence of the mutation on protease activity, further comprises:
inputting the sequence features and the structural features into the graph neural network, so as to obtain probability features related to activity change;
combining the probability features related to activity change with supplementary features from other prediction methods, and inputting the combined features into a decision tree model to obtain a classification of activity change; and
taking the mutation activity change labels as output targets, adjusting parameters of the prediction model.

29. The method according to claim 28, **characterized in that** said inputting the sequence features and the structural features into the graph neural network, so as to obtain the probability features related to activity change, comprises:
inputting the sequence features and the structural features into the graph neural network;
updating the features through matrix multiplication and aggregation operations using several layers of graph network structures, and then outputting the updated features;
merging the features output by the several layers of graph network structures, respectively;
compressing the features using a pooling operation; and
performing activity change classification on the features on the basis of a classification function of an output layer, and outputting the probability features related to activity change.

30. The method according to claim 21, **characterized in that** the prediction model is a classifier.

31. The method according to claim 21, **characterized in that** the graph neural network is a graph convolutional network or a graph attention network.

32. The method according to claim 28, **characterized in that** the decision tree model is a GBDT model.

33. The method according to claim 32, **characterized in that** the GBDT model is implemented using a LightGBM framework.

34. The method according to claim 21, **characterized in that** the step of acquiring the training set comprises:
acquiring experimental data from a public database as a training set; and
doubling the size of the training set by performing inversion processing on the experimental data from the public database.

35. A system for predicting the influence of a mutation on protease activity, **characterized in that** the system comprises:
an acquisition module, configured to acquire sequence information and three-dimensional structural information of a protease sample to be predicted; and
a processing module, configured to obtain a prediction result for activity change of the protease sample to be predicted after mutation by inputting the sequence information and the three-dimensional structural information of the protease sample to be predicted, wherein the processing module further comprises the following sub-modules:
a sequence feature sub-module, configured to perform feature extraction using a pre-trained model on the basis of the sequence information of the protease sample to be predicted, so as to obtain a sequence feature of the protease sample to be predicted;
a structural feature sub-module, configured to obtain a structural feature of the protease sample to be predicted on the basis of the three-dimensional structural information of the protease sample to be predicted; and
a model prediction sub-module, configured to perform prediction using a graph neural network-based prediction model on the basis of the sequence feature and the structural feature, so as to determine a prediction result for activity change of the protease sample to be predicted after mutation.

36. A computer-implemented system for predicting the influence of a mutation on protease activity, **characterized in that** the computer-implemented system implements the following operations by executing a computer program:
acquiring sequence information and three-dimensional structural information of a protease sample to be predicted;
obtaining a prediction result for activity change of the protease sample to be predicted after mutation by inputting the sequence information and the three-dimensional structural information of the protease sample to be predicted, wherein the operation further comprises the following sub-operations:
performing feature extraction using a pre-trained model on the basis of sequence information of a protease sample to be predicted, so as to obtain a sequence feature of the protease sample to be predicted;
obtaining a structural feature of the protease sample to be predicted on the basis of three-dimensional structural information of the protease sample to be predicted; and
performing prediction using a graph neural network-based prediction model on the basis of the sequence feature and the structural feature, so as to determine a prediction result for activity change of the protease sample to be predicted after mutation.

37. A non-transitory computer-readable storage medium for storing a computer program, **characterized in that** the computer program comprises instructions which, when executed by a processor of an electronic device, cause the electronic device to perform the method for predicting the influence of the mutation on protease activity according to any one of claims 1-20, or the method for constructing the prediction model for predicting the influence of the mutation on protease activity according to any one of claims 21-34.

38. A computer system, **characterized in that** the computer system comprises:
a processor;
a memory; and
a computer program, wherein the computer program is stored in the memory and configured to be executed by the processor, and the computer program comprises instructions for performing the method for predicting the influence of the mutation on protease activity according to any one of claims 1-20, or the method for constructing the prediction model for predicting the influence of the mutation on protease activity according to any one of claims 21-34.
